Europäisches Patentamt

European Patent Office

Office européen des brevets

(19)

(11) Veröffentlichungsnummer: **0 279 206**
**B1**

(12) EUROPÄISCHE PATENTSCHRIFT

(45) Veröffentlichungstag der Patentschrift:
24.10.90

(51) Int. Cl.⁵: **B67B 1/03**

(21) Anmeldenummer: **88100852.8**

(22) Anmeldetag: **21.01.88**

(54) Verfahren zur Sterilisation von Flaschenkorken.

(30) Priorität: **20.02.87 DE 3705422**

(43) Veröffentlichungstag der Anmeldung:
**24.08.88 Patentblatt 88/34**

(45) Bekanntmachung des Hinweises auf die Patenterteilung:
**24.10.90 Patentblatt 90/43**

(84) Benannte Vertragsstaaten:
**AT BE CH DE ES FR IT LI LU**

(56) Entgegenhaltungen:
**DE-A- 3 035 646**
**FR-A- 329 116**
**FR-A- 907 518**
**FR-A- 1 094 728**
**US-A- 2 388 753**

(73) Patentinhaber: **Schätzlein, Reinhold, Würzburger Strasse 6, D-8701 Eibelstadt(DE)**

(72) Erfinder: **Schätzlein, Reinhold, Würzburger Strasse 6, D-8701 Eibelstadt(DE)**

(74) Vertreter: **Fuchs, Richard, Kantstrasse 18, D-8700 Würzburg(DE)**

ACTORUM AG

## Beschreibung

Die Erfindung bezieht sich auf ein Verfahren zur Sterilisation von Flaschenkorken, insbesondre Korken für Wein-, Schaumwein- und Spirituosenflaschen, bei dem die Korken mittels einer sterilisierenden Flüssigkeit behandelt, z.B. getaucht oder besprüht und anschließend getrocknet werden.

Es ist bekannt, zur Sterilisation bzw. Keimfreimachung von Flaschenkorken für die oben bezeichneten Anwendungsfälle die Korken über eine gewisse Zeitspanne in chloriertes oder mit Wasserstoffperoxyd versetztes Wasser einzutauchen und nach dieser Behandlung zu trocknen. Wenn zwischen einer solchen Behandlung der Korken und ihrer Verwendung in Abfüllbetrieben eine größere Zeitspanne liegt, was die Regel ist und dadurch eine "Reinfektion" durch Mikroorganismen erfolgen kann, werden sie nochmals einer Sterilisationsprozedur unterworfen, die auch eine übliche sogenannte Schwefelsterilisation sein kann, bei der man Schwefeldioxyd auf die Korken einwirken läßt. Diese bekannten Sterilisationsverfahren weisen jedoch zahlreiche Mängel auf. So hat sich in der Praxis gezeigt, daß das Chlor oder Wasserstoffperoxyd in der Behandlungsflüssigkeit oder auch Schwefeldioxyd zu einer mindestens teilweisen Zerstörung der natürlichen Korkstruktur bzw. des Zellgewebes der Korken führen kann, mit der Folge, daß solche Flaschenkorken zu einer Leckage neigen (sog. Ausläufer). Hinzu kommt, daß die obigen bekannten Methoden auch in bezug auf ihren Sterilisationseffekt bis hin zum Weinabfüller und Weinkunden, die nicht selten Schimmilbildung am Korken feststellen können, nicht zufriedenstellend sind. Außerdem ist bekannt, daß durch die Sterilisation der Flaschenkorken mit chloriertem Wasser Trichloranisol entstehen kann, welches in äußerst negativer Weise die mit solchen Korken in Flaschen verschlossenen Getränke durch den sog. Korkmuff beeinflußt. Auch die Schwefelsterilisation der Korken kann zu Mufftönen bei den damit verschlossenen Getränken führen. Diese negativen Einflüsse schadeten in der Vergangenheit dem Ruf des Korkens als Flaschenverschluß dermaßen, daß Alternativverschlüsse, z.B. Schraubverschlüsse angewendet wurden, obwohl diese bei weitem nicht die Qualität und das Image von guten Flaschenkorken erreichen.

Der Erfindung liegt daher die Aufgabe zugrunde, ein hochwirksames Sterilisationsverfahren für Flaschenkorken zu schaffen, das keinen schädlichen Einfluß auf die natürliche Korkstruktur ausübt, so daß der Abdichteffekt der Flaschenkorken gewährleistet bleibt und welches ferner die Entstehung von Korkmuff bei Anwendung der Korken als Flaschenverschlüsse ausschließt.

Gemäß der Erfindung wird obige Aufgabe dadurch gelöst, daß die Sterilisation der Flaschenkorken mittels ozonisiertem Wasser und eine Nachsterilisation mittels einer ozonisierten Siliconemulsion mit einem Anteil von wenigstens 1 mg Ozon pro Liter Wasser bzw. Emulsion erfolgt, das bzw. die während der Behandlung der Korken auf eine Temperatur unter etwa 30°C gehalten wird. Man erreicht mit dieser Sterilisationsmethode eine Schonung der Korkstruktur bzw. des Zellgewebes der Korken und bewahrt somit die abdichtende Eigenschaften des Korkmaterials, was zur Folge hat, daß solche Flaschenkorken im Gebrauch auch keine Schimmelbildung an den Außenseiten aufweisen, die bei nach bekannten Sterilisationsverfahren behandelten Flaschenkorken aufgrund von Feuchtigkeitseinwirkung häufig zu beobachten ist. Nach dem erfindungsgemäßen Verfahren sterilisierte Flaschenkorken weisen also praktisch keine Ausläufernegung auf, besitzen eine höhere Sterilität als die mit bekannten Methoden behandelten Flaschenkorken und schließen bei ihrer Anwendung als Flaschenverschlüsse vor allem die Entstehung eines sog. Korkmuffes bei den damit verschlossenen Getränken aus. Ein weiterer Vorteil besteht darin, daß nach der erfindungsgemäßen Sterilisation der Flaschenkorken keine Nachspülung derselben erforderlich ist und die Korken sofort getrocknet und verpackt werden können. Nach der Behandlung der Korken zerfällt nämlich das Ozon vollständig und es verbleiben keinerlei Rückstände, wie z.B. bei der bekannten Sterilisation mit chloriertem Wasser. Durch die Erfindung erlangt der Korken, als der seit Jahrhunderten natürlichste Flaschenverschluß seine Bedeutung insbesondere für die Weinwirtschaft zurück, denn ein guter und chemisch neutraler Naturkork trägt zum Image und zur Qualität des Weines als dessen letzte Bearbeitungsstufe erheblich bei.

Bevorzugt werden die Flaschenkorken in das ozonisierte Wasser und in die ozonisierte Siliconemulsion getaucht. Ein Aufsprühen der ozonisierten Flüssigkeiten auf die Korken ist nur bedingt wirksam, liegt jedoch gleichfalls im Rahmen der Erfindung. Die Einwirkdauer der ozonisierten Flüssigkeiten auf die Flaschenkorken hängt vom jeweiligen Ozongehalt ab und beträgt z.B. beim Minimum von 1 mg Ozon pro Liter Wasser bzw. Emulsion ca. 1/4 Stunde, um die geforderte hohe Sterilisationsqualität zu erreichen.

Die Anwendung der ozonisierten Siliconemulsion bringt den Vorteil, daß die Korken gleichzeitig imprägniert werden. Dabei kann die Siliconemulsion, unterstützt durch das fein gelöste Ozon, besser in die Korkstruktur, d.h. vor allem tief in die Markstrahlen und Poren der Korken eindringen. Die wasserabweisende Siliconemulsion dichtet auf diesem Wege die Korken bzw. das mit diesen verschlossene Getränk weitestgehend ab, verzögert die Durchfeuchtung der Korken und ist zugleich das notwendige Gleitmittel beim Verschließen von Flaschen.

Ausgestaltungen der Erfindung gehen aus den Unteransprüchen hervor. So wird bevorzugt als Sterilisationsflüssigkeit ozonisiertes Wasser bzw. eine ozonisierte Siliconemulsion mit einem Anteil von 2–3 mg Ozon pro Liter Wasser bzw. Emulsion verwendet. In diesem Fall genügt nämlich eine Einwirkzeit der ozonisierten Flüssigkeit auf die Korken von nur etwa 3 Minuten, was für die Wirtschaftlichkeit des erfindungsgemäßen Verfahrens spricht.

Nach noch einer Weiterbildung der Erfindung wird die Temperatur des onzonisierten Wassers bzw. der ozonisierten Siliconemulsion während der Behandlung der Korken auf ca. 15°C gehalten. Bei dieser Temperatur wird ein besonders starker Sterilisationseffekt erreicht.

Wenn nach noch einer Weiterbildung der Erfindung die Flaschenkorken im eingetauchten Zustand bewegt oder umgekehrt das ozonisierte Wasser und die ozonisierte Siliconemulsion bei eingetauchten, ruhenden Flaschenkorken umgewälzt werden, kann das Ozon noch besser in die Poren und Markstrahlen der Flaschenkorken eindringen, was den Sterilisationseffekt weiter verbessert.

Im folgenden wird ein bevorzugtes Ausführungsbeispiel der Erfindung beschrieben. Zunächst ist festzuhalten, daß das zur Ozonisierung vorgesehene Wasser frei von organischen Verbindungen und Chlor sein muß und daher einer entsprechenden Aufbereitung bedarf. Der ph-Wert des Wassers sollte bei 7 und die Härtegrade desselben sollten unter 20° dH, vorzugsweise bei 5 - 7° dH liegen. Die Einrichtung zur Durchführung des erfindungsgemäßen Sterilisationsverfahrens muß, soweit sie mit dem ozonisierten Wasser bzw. der ozonisierten Siliconemulsion in Berührung kommt, aus ozonbeständigen Werkstoffen hergestellt sein. Hierunter fallen vor allem das Gefäß zur Aufnahme der ozonisierten Flüssigkeit und das Behältnis mit der Tragevorrichtung für die zu tauchenden Flaschenkorken.

In einer ersten Stufe werden die keimfrei zu machenden Flaschenkorken in ozonisiertes Wasser getaucht und darin bewegt. Die Temperatur des ozonisierten Wassers wird dabei konstant auf ca. 15° C gehalten und der Ozonanteil wird zur Erreichung eines gleichbleibenden Sterilisationseffekts laufend kontrolliert, wobei dieser im bevorzugten Ausführungsbeispiel 2 - 3 mg Ozon pro Liter Wasser beträgt. In diesem Fall genügt eine Behandlungsdauer der Flaschenkorken von etwa 3 Minuten. Nach der Herausnahme aus dem ozonisierten Wasser werden die Flaschenkorken, ohne daß es einer Nachspülung bedarf, sofort z.B. in einer Warmluftströmung getrocknet und anschließend verpackt. Da zwischen dieser ersten Sterilisation und der Verarbeitung der Flaschenkorken in einem Abfüllbetrieb in der Regel eine größere Zeitspanne liegt, in der ein erneuter Befall der Korken durch Mikroorganismen erfolgen kann, werden die Korken kurz vor dem Verkauf an Abfüllbetriebe nachsterilisiert. Hierzu werden die Flaschenkorken in eine ozonisierte Siliconemulsion getaucht, die gleichfalls 2 - 3 mg Ozon pro Liter Emulsion enthält und auf eine Temperatur von 15° C gehalten wird. Die Einwirkzeit beträgt auch in diesem Fall ca. 3 Minuten. Anschließend werden die Korken wieder z.B. in einem Warmluftstrom getrocknet, danach bedruckt, luftdicht verpackt und an die Abfüllbetriebe versandt.

## Patentansprüche

1. Verfahren zur Sterilisation von Flaschenkorken insbesondere Korken für Wein-, Schaumwein- und Spirituosenflaschen, bei dem die Korken mittels einer sterilisierenden Flüssigkeit behandelt, z.B. getaucht oder besprüht und anschließend getrocknet werden, dadurch gekennzeichnet, daß die Sterilisation der Flaschenkorken mittels ozonisiertem Wasser und eine Nachsterilisation mittels einer ozonisierten Siliconemulsion mit einem Anteil von wenigstens 1 mg Ozon pro Liter Wasser bzw. Emulsion erfolgt, das bzw. die während der Behandlung der Korken auf eine Temperatur unter etwa 30°C gehalten wird.

2. Verfahren nach Anspruch 1, dadurch gekennzeichnet, daß als Sterilisationsflüssigkeit ozonisiertes Wasser bzw. eine ozonisierte Siliconemulsion mit einem Anteil von 2–3 mg Ozon pro Liter Wasser bzw. Emulsion verwendet wird.

3. Verfahren nach Anspruch 1 oder 2, dadurch gekennzeichnet, daß die Temperatur des ozonisierten Wassers bzw. der ozonisierten Siliconemulsion während der Behandlung der Korken auf ca. 15°C gehalten wird.

4. Verfahren nach einem der Ansprüche 1–3, dadurch gekennzeichnet, daß die Flaschenkorken im eingetauchten Zustand bewegt oder umgekehrt das ozonisierte Wasser und die ozonisierte Siliconemulsion bei eingetauchten, ruhenden Flaschenkorken umgewälzt wird.

## Claims

1. Method for sterilisation of cork-stoppers especially cork-stoppers for wine, sparkling wine and spirits by treating the stoppers with a sterilized liquid for example dived or sprayed and subsequently dryed, characterized in that the sterilization of cork-stoppers is carried out by ozonic water and a poststerilization is carried out by a ozonic silicon-emulsion with a share of at least 1 mg ozon per liter water respectively emulsion, which is kept below a temperature of perhaps 30°C during the treatment of the cork-stoppers.

2. Method according to claim 1, characterized in that ozonic water respectively a ozonic silicon-emulsion with a share of 2–3 mg ozon per liter water respectively emulsion is used as a sterilization-liquid.

3. Method according to claim 1 or 2, characterized in that the temperature of the ozonic water respectively the ozonic silicon-emulsion is kept at about 15°C during the treatment of the cork-stoppers.

4. Method according to one of the claims 1–3, characterized in that the cork-stoppers are moved, when they are dived or reversed the ozonic water and the ozonic silicon-emulsion is rolled around, when the cork-stoppers are dived and rested.

## Revendications

1. Procédé pour stériliser des bouchons de bouteilles, en particulier des bouchons pour des flacons ou bouteilles de vin, de vins mousseux et de spiritueux, dans lequel les bouchons sont traités, par exemple par immersion ou par aspersion, à l'aide d'un liquide stérilisant et sont ensuite séchés, procédé caractérisé en ce qu'on effectue la stérilisation des bouchons à l'aide d'une eau ozonisée et en effectuant une post-stérilisation réalisée à l'aide d'une émulsion de silicone ozonisée, avec une proportion d'au moins 1 mg d'ozone par litre d'eau ou d'une émulsion de silicone ozonisée, avec une proportion d'au moins 1 mg d'ozone par litre d'eau ou d'émulsion, l'eau ou l'émulsion étant maintenue pendant le traitement des bouchons à une température inférieure à 30°C environ.

2. Procédé selon la revendication 1, caractérisé en ce qu'on utilise comme liquide pour la stérilisation de l'eau ozonisée ou une émulsion de silicone ozonisée présentant une proportion de 2 à 3 mg d'ozone par litre d'eau ou, d'émulsion.

3. Procédé selon la revendication 1 ou 2, caractérisé en ce qu'on maintient, pendant le traitement des bouchons, au voisinage d'environ 15°C la température de l'eau ozonisée ou de l'émulsion de silicone ozonisée.

4. Procédé selon l'une des revendications 1 à 3, caractérisé en ce qu'on agite ou déplace les bouchons quand ils sont immergés ou bien, inversement, on soumet l'eau ozonisée et l'émulsion de silicone ozonisée à une circulation par recyclage quand les bouchons de bouteilles sont immergés au repos.